# EUROPEAN PATENT APPLICATION

(11) **EP 3 407 168 A1**
(43) Date of publication of application: **28.11.2018**
(21) Application number: 18173610.9
(22) Date of filing: 22.05.2018
(51) Int. Cl.: G06F 3/01, A61B 5/00

(54) **DETERMINING FULL-BODY POSE FOR A VIRTUAL REALITY ENVIRONMENT**

(30) Priority: 25.05.2017 US 201762511004 P
(71) Applicant: THOMSON LICENSING, 92130 Issy les Moulineaux (FR)
(72) Inventor: HAMIDI-RAD, Shahab, Los Altos, CA California 94022 (US); LYONS, Kent, Los Altos, CA California 94022 (US); PUSHPARAJA, Akshay, Los Altos, CA California 94022 (US); YAO, Zijun, Los Altos, CA California 94022 (US); AGARWAL, Gaurav, Los Altos, CA California 94022 (US); ZHANG, Alan, Los Altos, CA California 94022 (US); KANCHINADAM, Teja, Los Altos, CA California 94022 (US); KHURANA, Rushil, Los Altos, CA California 94022 (US)
(74) Representative: Vidon Brevets & Stratégie

(57) **Abstract**

A method to determine a body pose of a user in a virtual reality or augmented reality system includes acquiring sensor data from a plurality of sensors in a garment worn by a user. The sensor data is processed to generate a processed sensor data set, wherein the processed sensor data set is scaled for the size of the user. The processed sensor data set is converted to a pose data set. The pose vector data set is then used by a viewer device to render the body pose of the user.

## Description

### CROSS REFERENCE TO RELATED APPLICATIONS

This application claims the benefit, under 35 U.S.C. § 119 of US provisional application 62/511,004 filed on May 25, 2017. The US provisional application is herein incorporated by reference in its entirety for all purposes.

### FIELD

The present principles relate to virtual reality systems, specifically, it relates to determining a body pose of a user of a virtual reality system.

### BACKGROUND

In a virtual reality (VR) environment, if one wants to showcase a user to other users (or to herself), it is not possible to showcase an exact body pose of the user as in the real world. Generally, in such an environment, users are disembodied. One way to deal with this is to fake the body measurements of the user and showcase her in the virtual or augmented reality environment. An approximation of body pose can be used or an avatar with a selected body pose can be used to depict the user to others. But this is not the ideal way to deal with the problem of how to accurately determine a body pose. Current solutions include using special gloves to show the hand positions. But it does not accurately display all body parts in a body pose representation. A more accurate determination of body pose is desirable for virtual reality or augmented reality systems use.

### SUMMARY

This summary is provided to introduce a selection of concepts in a simplified form as a prelude to the more detailed description that is presented later. The summary is not intended to identify key or essential features, nor is it intended to delineate the scope of the claimed subject matter.

In one embodiment, a method to render a body pose of a user includes acquiring sensor data from a plurality of sensors worn by a user and processing the acquired sensor data to generate a processed sensor data set, wherein the processed sensor data set is scaled for the size of the user. The method then converts the processed sensor data set into a pose data set. The pose data set is translated into a format for display on a viewer device. The formatted pose data set is transmitted to a viewer that renders the body pose of the user.

In one embodiment, an apparatus to provide information for a body pose of a user includes a receiver to receive sensor data from a plurality of sensors worn by the user. A processor processes the received plurality of sensor data and provides a processed sensor data set scaled to the size of the user. A machine learning model converts the processed sensor data set into a pose data set. A transmitter provides the pose data set to a display in a format that can render the body pose of the user.

Additional features and advantages will be made apparent from the following detailed description of illustrative embodiments which proceeds with reference to the accompanying figures. The drawings are for purposes of illustrating the concepts of the disclosure and is not necessarily the only possible configuration for illustrating the disclosure. Features of the various drawings may be combined unless otherwise stated.

### BRIEF DESCRIPTION OF THE DRAWINGS

The foregoing summary, as well as the following detailed description of illustrative embodiments, is better understood when read in conjunction with the accompanying drawings, which are included by way of example, and not by way of limitation with regard to the present principles. In the drawings, like numbers represent similar elements.
Figure 1 is a first depiction of a virtual reality system having aspects of the disclosure;
Figure 2 is a second depiction of a virtual reality system having aspects of the disclosure;
Figure 3 is a virtual reality processor functional block diagram having aspects of the disclosure;
Figures 4a depicts an example wearable sensor set configuration having aspects of the disclosure;
Figure 4b depicts a an example sensor set electronics having aspects of the disclosure;
Figure 5 depicts an example flow diagram for the VR processor having aspects of the disclosure;
Figure 6a is a first depiction of training set poses;
Figure 6b is a first depiction of training set poses;
Figure 7 is a virtual reality processor block diagram having aspects of the disclosure;
Figure 8 contains Table 1;
Figure 9 contains Table 2; and
Figure 10 contains Table 3.

### DETAILED DISCUSSION OF THE EMBODIMENTS

In the following description of various illustrative embodiments, reference is made to the accompanying drawings, which form a part thereof, and in which is shown, by way of illustration, how various embodiments may be practiced. It is to be understood that other embodiments may be utilized and structural and functional modification may be made without departing from the scope of the present principles.

The configuration disclosed herein is useful to display a full body pose of a user which reflects their actual body pose. This discussion herein teaches using the measurements captured by one or more smart wearable items, such as smart clothing, to show users in a VR environment. The full body pose of the users could be rendered more accurately using the measurements provided by the smart wearable item.

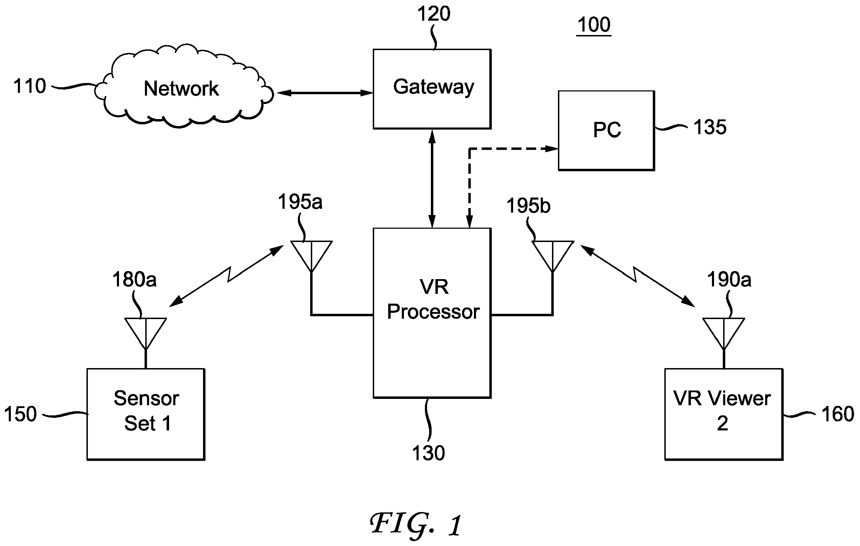

Figure 1 depicts a virtual reality or augmented reality environment 100 wherein the body pose measurement improvement of this disclosure may be used. Although explained in terms of a virtual reality use, the current disclosure applies equally well to an augmented reality use. Figure 1 depicts a virtual reality (VR) processor 130 connected to a gateway 120, further connected to a network 110. The gateway acts as an interface for the VR processor to gain access to a wide area network (WAN), such as the internet or other on-line resource provided by a service provider. In the example environment instance shown in Figure 1, a VR viewer, such as a headset for a VR user is displaying information derived from a sensor set 150. In the example, data from a sensor set 150 is transmitted via antenna 180a to antenna 195a of the VR processor 130. The transmission link shown may be RF acoustic, infrared, or any other short distance wireless transmission. Alternately, a wired transmission instead of a wireless transmission may be used. However, in the example, a WiFi compatible interface is contemplated.

In one aspect of the disclosure, raw sensor information is acquired by the VR processor 130 from the sensor set 150 and transformed into pose information related to the sensor set. For example, the pose information could be a physical pose of a player, such as a human player, acting in the VR environment, that is wearing the sensor set. The pose information is transmitted from the VR processor via antenna 195b to the VR viewer 160 via antenna 190a. The pose information is then converted into a display that is for use by another user or player of the VR system. In this manner, the pose of the person wearing the sensor set 150 is rendered and visible to the other user of the VR system in real-time in near-real-time. In the environment 100, a personal computer (PC) 125 may optionally be used to assist in the configuration of either that VR processor 130 or the gateway 120.

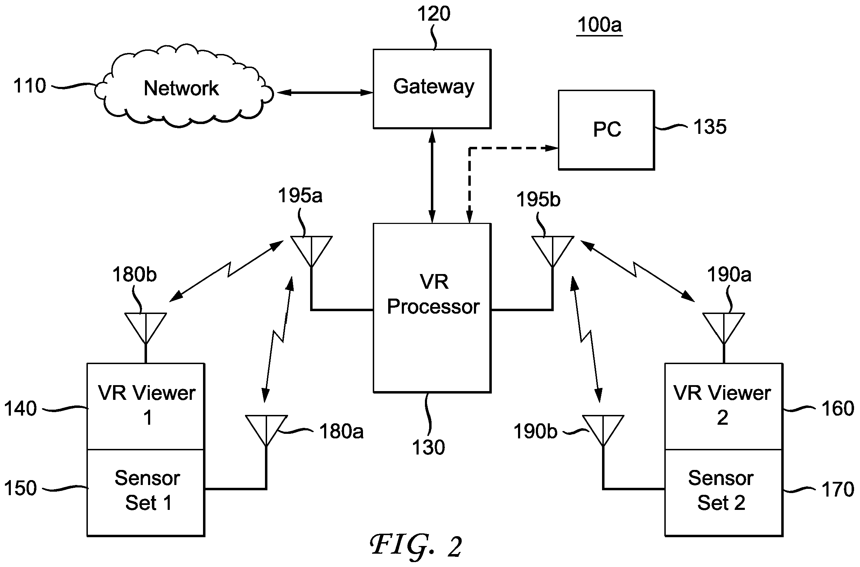

Figure 2 is a representation of the environment of Figure 1 where two users or players in the VR environment 100a are capable of viewing the body pose of each other. As before a first sensor set 150 is worn by a first user and first sensor set data is transmitted via antenna 180a to VR processor 130. The sensor data from the first user is transformed to pose information of the first user in the VR processor 130 and then communicated to the VR viewer 160 via antennas 195b and 190a. In a complementary flow, a second sensor set 170 is worn by a second user and the second sensor set data is transmitted via antenna 190b to VR processor 130. The sensor data from the second user is transformed to pose information of the second user in the VR processor 130 and then communicated to the VR viewer 140 of the first user via antennas 195a and 180b. In the environment of Figure 2, both a first and second user may each see an accurate rendering of a body pose of the other user by using the principles of the present disclosure. In one aspect of the disclosure, the size and scales of subjects (users) in the real world are accurately replicated in the virtual world.

Measurements from the sensors in the sensor set, which part of a smart wearable item, are captured by electronics that are part of the wearable item. The data from the sensors may be used for activity tracking of the wearer. Using smart clothing, accurate body measurements of the user can be obtained using certain measuring techniques such as segmentation. The body pose of the user can be determined using these measurements and render them in the virtual environment. For example, as the user moves his body to a new pose, the sensors on the smart clothing stretch/shrink/bend and hence create a new set of measurements. The new set of measurements are then transformed to a new pose for the user in the VR environment.

For example, a strain sensor (such as those commercially available) can measure stretch, pressure, bend, and shear. When embedded properly in a smart wearable, such as clothing, these sensors can provide measurements that help render a person's current pose more accurately. For each smart wearable, based on the position of its sensors, a computer model can be trained, using machine learning algorithms for example, that receives all measurements from all the sensors and calculates a current pose of the user which is then rendered by the VR device.

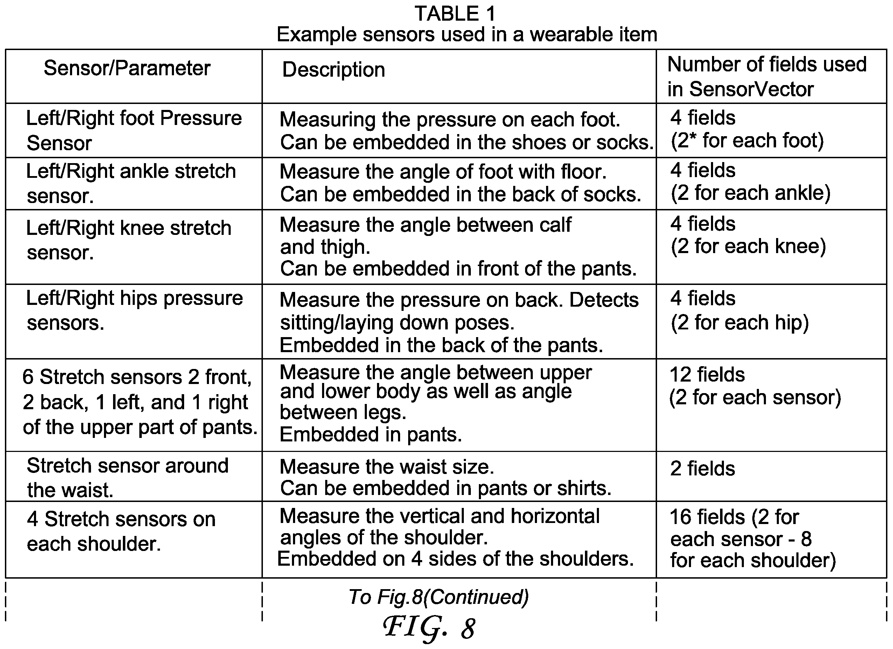

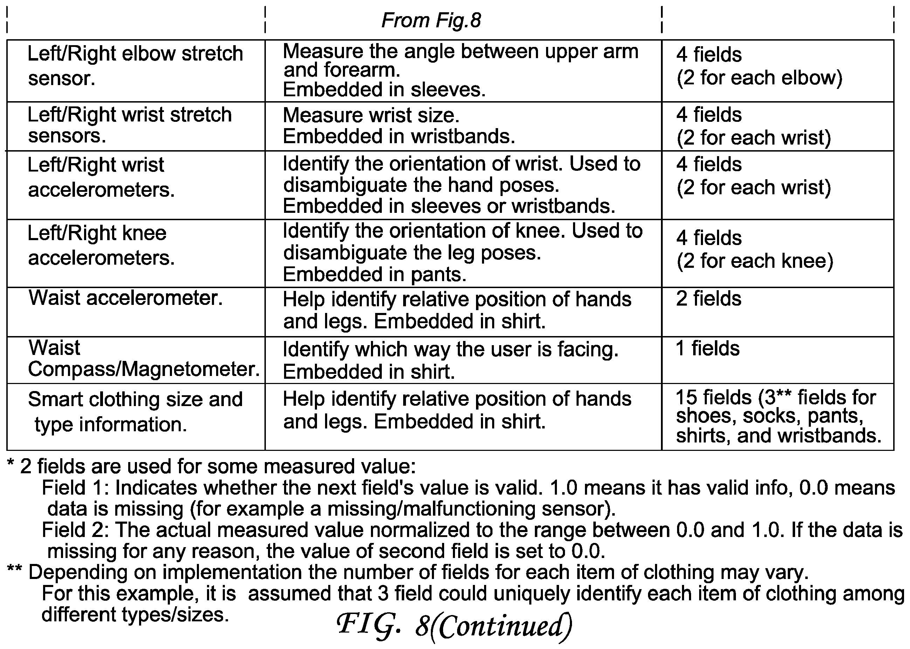

The smart wearable, containing the sensor set, is equipped with several sensors measuring different types of parameters. This could include but not limited to stretch sensors, pressure sensors, magnetometers (in combination with permanent magnets), accelerometers, level indicators, and the like. The parameters measured by these sensors are translated to information that can be used to accurately render a person's body in the VR world. Figure 8 includes Table 1 as an example of sensors (sensor set) used in an example smart wearable item, such as clothing. In such a wearable item, the sensors are sewn into or weaved into the fabric of the wearable item and interconnected as described later herein below.

Not all sensors are available in all smart wearable items. Specifically, the sensors measuring size of body parts may not be included in all types of smart wearable items. Assuming the body size of the VR users do not change frequently, this information can be measured (or manually entered) once and saved in the user profile metadata for each specific user. Example body size can include one or more of overall height, limb length, waist size, and the like for scaling purposes. In one embodiment, this user information may be entered via the personal computer 135 of Figures 1 and 2.

When a new smart wearable item is being introduced to the system of Figures 1 and 2, the information related to all sensors on the wearable item, such as clothing, is added to the VR processors' 130 data base of sensors. This information includes the location and type of the sensor and the metadata about the range of measured parameters, etc. In a typical embodiment, the VR processor has already been trained with many different known types of sensors on different locations. After registering all the smart wearable items and the related sensors, the VR system knows about all the sensors and types of information it receives from them. The registration process also assigns a unique identifier (ID) to each sensor.

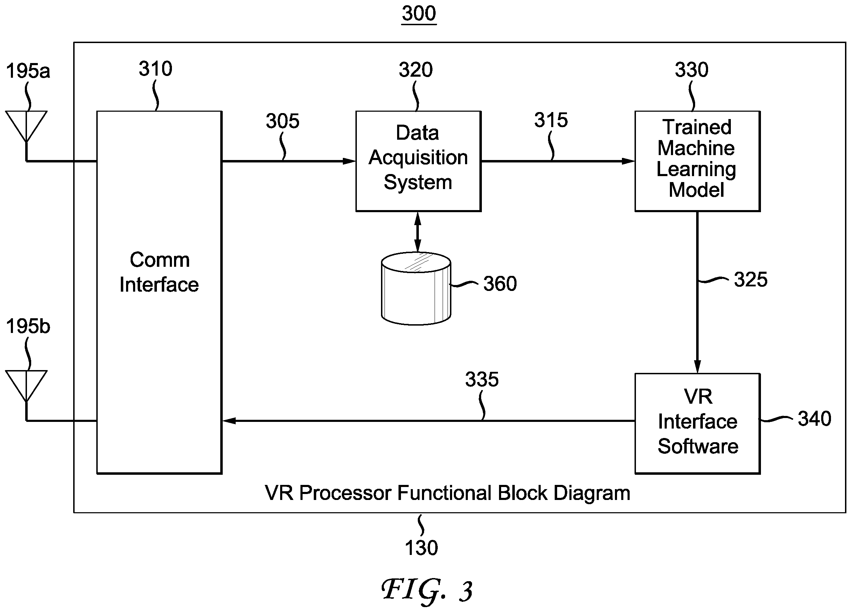

Figure 3 is a functional block diagram 300 of a typical VR processor 130 according to the present principles. A communication interface 310 supports the functioning of antennas 195a and 195b. It is noted that, as in many WiFi systems, one or a plurality of antennas may be used. The number of the various antenna depictions in the various figures of the disclosure are exemplary as understood by one of skill in the art. Other configurations are possible within the scope of the functionality of the current disclosure.

Raw sensor data is derived from a sensor set, such as sensor set 150, and is transmitted via antenna 180a of Figures 1 and 2. Antenna 195a receives the sensor set information and communication interface 310 converts the modulated WiFi or other received formatted signal into digital data. The digital data is transmitted to data acquisition system 320 in the form of a sensor identifier and sensor value pair (ID, Value) via connection 305. When using the system 300, a data acquisition system 320 receives pairs of (ID, value) from each sensor from all smart wearable items at different time periods. The transfer of data from sensors to the data acquisition system may differ depending on the types of sensors. For example, a sensor may wait to be polled for the measured data, another sensor may keep measuring and sending the data using a period of time appropriate for the sensor set. Note that the smart wearable itself does not do any data processing. The smart wearable functions to acquire sensor data (measured parameters) and pass the sensor measurement information to the data acquisition system 320 of the VR processor 130.

Data acquisition and preprocessing are performed on the ID, Value pairs of sensor data in the data acquisition system 320. The way the sensor information pair readings are collected can vary from sensor to sensor. The data acquisition system 320 makes these differences transparent to the rest of the system. For example, a sensor may be polled periodically for the new readings and the other could send new information automatically as they become available. The data acquisition system 320 can perform error detection/correction on received measured data if the data is received with error correction capability. In that instance, and the acquisition system can also organize and mark missing information. Missing data can be replaced with information from user profile when possible. For example, if some body part sizes are missing from the measured information, their value can be extracted from the user profile metadata stored in a user profile database 360.

The data acquisition system 320 performs the preprocessing on the received raw sensor data. This preprocessing includes scaling and normalization of the measured information. For example, for most machine learning algorithms, it is preferred that the input vector data elements be in the range of 0.0 to 1.0 (or -1.0 to 1.0). The data acquisition system provides the scaling and ranging of the data. Periodically (for example every 100ms), the data acquisition system 320 packs all of the received and processed information into a vector format termed SensorVector or SensorVector data. The SensorVector data, also known as processed sensor data, is provided to a trained model 330 via connection 315.

For each user, personalized user profile information about his/her body size is gathered when they sign up to the system for the first time via PC 135. The information can be gathered automatically when they wear the smart clothing for the first time. This is useful when the smart clothing is equipped with all the sensors needed to measure body parts. The body size information may also be entered manually to the system by the user. Once this information is stored with the user profile, the data acquisition system can embed this information into the specified fields in the SensorVector data (processed sensor data set) if the measured values are not available. This process can aid in proper scaling of the raw sensor data while forming the SensorVector data (processed sensor data set).

In one embodiment, the SensorVector data (processed sensor data) is a fixed size vector; a fixed-length 1-dimensional array of floating point numbers. For each measured value, there are two entries in this vector. The first entry indicates the existence and validity of the measured value. This can be 0 (missing) or 1 (valid). The second entry contains the normalized value of the actual reading for that specific sensor. In addition, some other data is also included in the SensorVector data such as the metadata concerning smart wearable type and size for each wearable item.

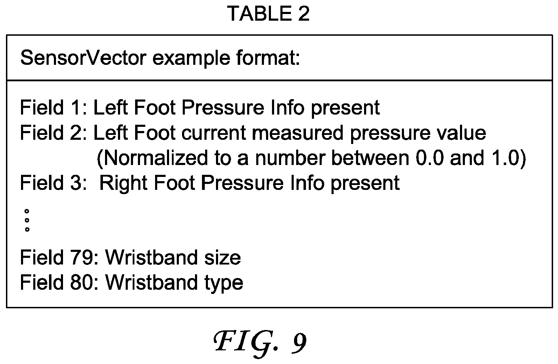

Table 2 in Figure 9 shows an example of SensorVector data format definition. In this example, the SensorVector data has 80 fields. As such, the SensorVector data is an array of 80 floating point numbers between 0.0 and 1.0. The fields can be labeled using Table 2.

As new types of smart wearables with more sensors become available, the old training data needs to be migrated to a new format that has more space for storing new sensor data. This SensorVector data format migration process is fast and easy. The old sensor vectors are extended and all new sensor info are marked as missing. This easy migration method allows re-using old data such that training information need not be recreated. Training of the machine learning model is discussed later herein.

In Figure 3, the data acquisition system 320 provides all the data received from the sensors to a pre-trained machine learning model 330. The data is sent in the form of an n-dimensional SensorVector (processed sensor data). Each element in SensorVector contains the information received from each sensor. The trained model 330 creates an output vector termed PoseVector that defines the current pose of the user having the sensor set 150 of a wearable item. PoseVector is also known as PoseVector data or simply pose data. The elements in the PoseVector contain information such as size of body parts and the angles between them which are eventually converted into pose information for the particular viewer and rendered by the VR system in a viewer 160, such as in a 3-D graphical interface or other display device.

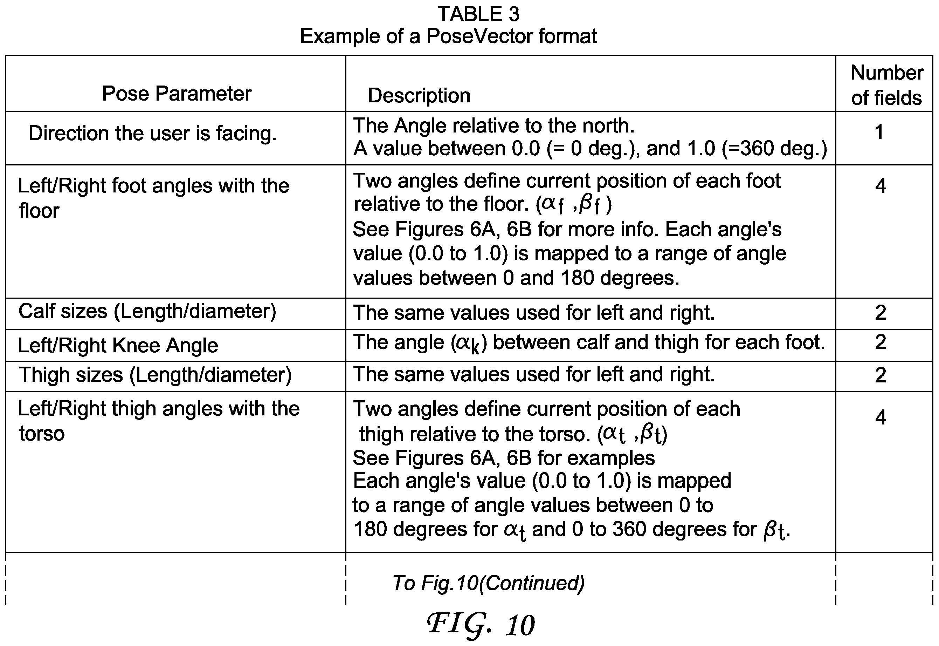

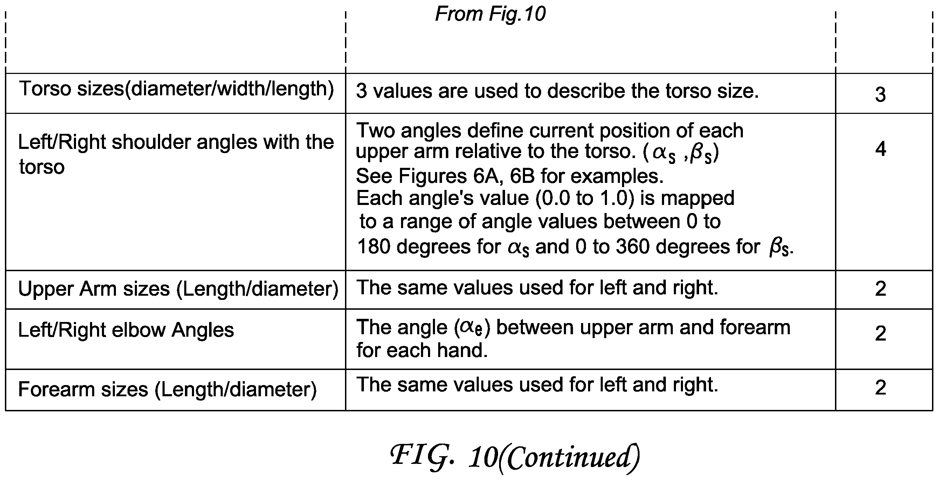

A pre-trained model 330 is used in real-time to convert each SensorVector to a corresponding PoseVector. The PoseVector contains information needed to accurately render the user's body in the VR world. One example PoseVector format is defined in Table 3 of Figure 10. This definition of PoseVector (pose data) uses a 28-dimensional vector to define each pose. Please note that this is only a simple exemplary definition for the PoseVector (pose data) which may not cover all possible human poses (For example, handstand, or while in the air as the user jumps up) But it is obvious that by adding more parameters to the PoseVector one can cover all possible poses.

The pose data are fed to a VR Interface Software 340 via connection 325 that converts the PoseVector (pose data) information to the format recognized by the particular VR viewer 160 vendor being used. New versions of VR Interface Software 340 are implemented for each new VR viewer 160, 140 vendor using the API and documentation provided by or with the VR viewer.

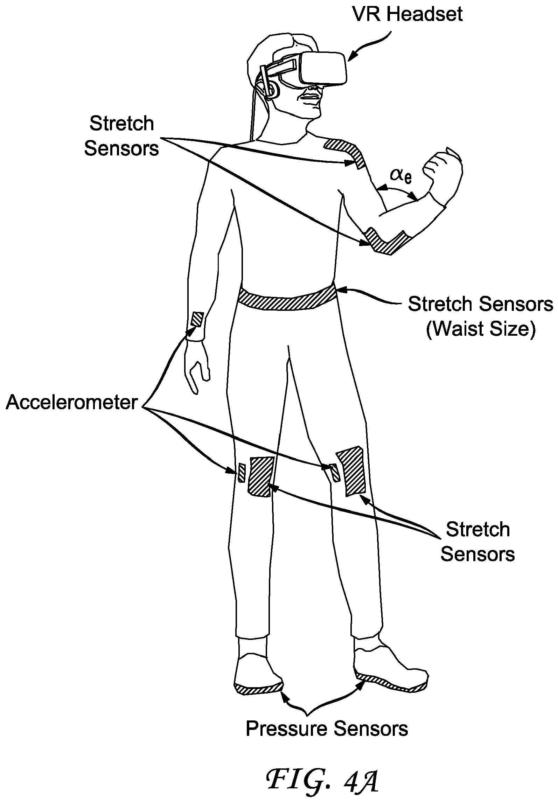

Figure 4a depicts one embodiment of a sensor set incorporated into a wearable item. In the example, the wearable item is a shirt, pants and shoes combination of wearable items. Each item may have different sensors to accommodate the measurement of parameters that, when processed, can produce a PoseVector (pose data) indicative of the body pose of the wearer. Also shown in Figure 4a is a depiction of the user/wearer having a VR headset. This is needed only if the User is to see others, or himself, in the VR environment.

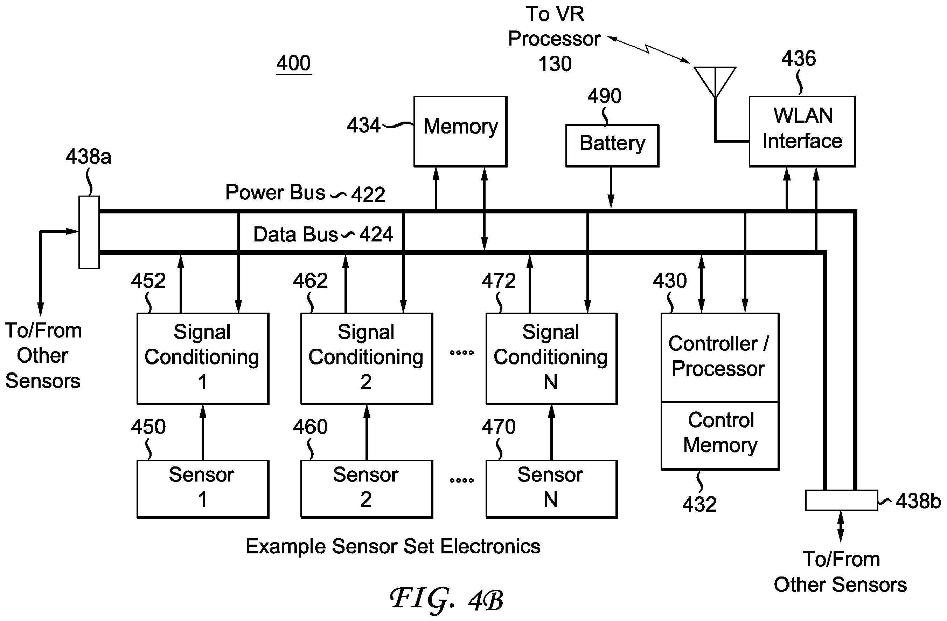

Figure 4b is an example configuration for a wearable item electronics 400 having a sensor set. As described before, such a wearable item may have the electronic components woven or sewn into the fabric of a wearable item. Sensors 450, 460, and 470 represent up to N sensors. Each may or may not have an associated signal conditioning unit 452, 462, and 472 respectively. The signal conditioning unit may be part of the sensor or may be separate and may include such functions as A/D conversion, voltage scaling, limiting, and the like. Data from a sensor is available on a data bus 424. In the embodiment of Figure 4b, a controller/processor 430 having control memory 432 for program memory is useful to poll the sensors and store the sensor data in memory 434 as needed. At an appropriate time, the collected data is transmitted to the VR processor 130 via a WLAN interface 436. Power for the electronics of a wearable item may be provided via a battery 490 or similar power source whose energy is distributed via power bus 422. Connectors 438a and 438b provide the flexibility of the electronics to be expandable for the interconnection of additional sensors and/or other wearable items to assist in the configuration of a full body sensor set as shown in Figure 4a.

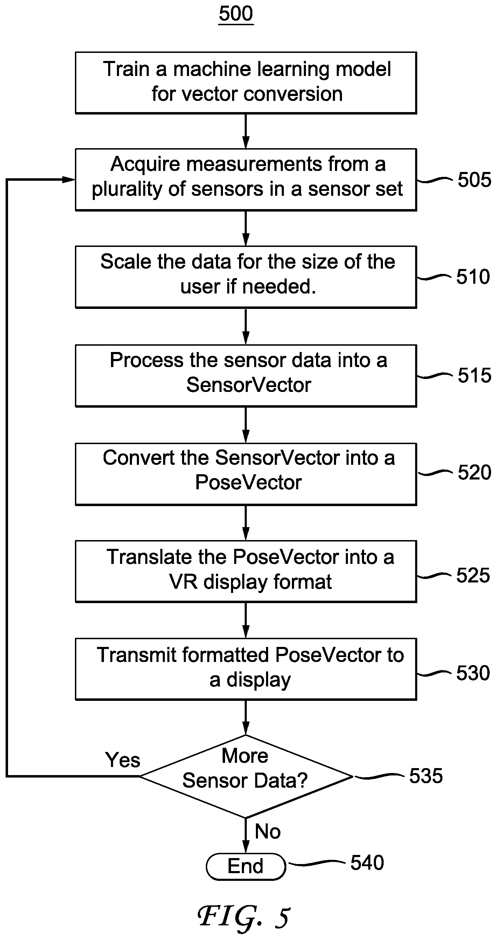

Figure 5 is a flow diagram 500 of the method performed by the VR processor 130. The flow assumes a trained model for the conversion of a SensorVector (processed sensor data) to PoseVector (pose data). Once trained, the process of acquiring data and providing an output suitable for a VR viewer is realized. Thus, step 501, training a vector conversion model, need not be performed each time a data set for the VR rendering is made. At step 501, a machine learning model is trained to perform a SensorVector (processed sensor data) to PoseVector (pose data) conversion. Once trained, the conversion model 330 may be used repetitively without re-training.

At step 505, data measurements from a sensor set of a wearable item are acquired. In one embodiment, the acquisition includes receiving sensor ID, Value pairs for each sensor in the sensor set. It is preferred that an entire sensor set of data be obtained, however, missing sensor data can be tolerated. The sensor data is acquired via an RF communication interface or equivalent and may use a formatting scheme such as that available through the use of WiFi.

At step 510 the received sensor data set is in ID, Value pairs and may be scaled and/or normalized for ease of processing. For example, the raw sensor data set may be scaled to substantially represent the size of the user. In one example instance, the scaling of a user representation in a viewer is to be the size of the user when viewed in a virtual or augmented reality environment with other scaled users. Other users are also scaled to their physical body measurements such that all users appear in physical size relation with respect to each other in the viewer. Such sizing information for a user may be available via a database in the VR processor. At step 515, the processed sensor set data is generated as a SensorVector data set (a set of processed sensor data) for further processing. Time-tagging the processed data set may be performed but is optional. Such time-tagging can be useful to separate processed sensor data sets from one another and keep the sensor data sets in an ordered sequence for display. Other methods of separation of processed data sets may also be used such as a simple numerical indexing. At step 520, the SensorVector (processed sensor data) is converted into a PoseVector data set (pose data set) using the previously trained machine learning model 330. SensorVector data (processed sensor data) that does not exactly match correlated sensor vector training data supplied to the learning model can be interpolated by the VR processor to provide a PoseVector (pose data) from SensorVector data (processed sensor data). At step 525, the PoseVector data (pose data) set is translated into a format compatible with a viewing device, such as a VR display device or other display. In one embodiment, the pose data is translated to a digital format compatible with a digital input data format used by a viewer device. Such a viewer device can be any device used to display a virtual reality or augmented reality rendering. A viewer device can be any of a wearable visor, goggles, glasses, and the like, or a display screen known in the art. The format translation of step 525 allows the pose data to be rendered by a viewer device (the wearable headgear or other display apparatus). At step 530, the PoseVector data set (pose data set) is transmitted to a display device, such as a VR display device. Thus, using the above steps, a body pose of a user may be generated displayed on a display device. One example of a VR display device is a VR headset worn by a user of a VR system. At step 535, if there is more sensor data available, the process repeats at step 505. If no additional sensor data is available, then the process 500 can end at step 540.

Returning to step 501, which does not have to be practiced in order to repetitively obtain a body pose after training the machine learning developed model 330, the training steps are outlined. To train the model 330, users with a different body size compared to others are asked to wear different types of smart wearables and go through a list of predefined poses. These predefined poses are considered training samples for the machine learning algorithm of the model 330. For these samples, both the SensorVector (processed sensor data) and PoseVector (pose data) are known. The specific SensorVector for the particular user for the specific pose selected is created using the data acquisition system because the PoseVector is already known for each one of predefined poses.

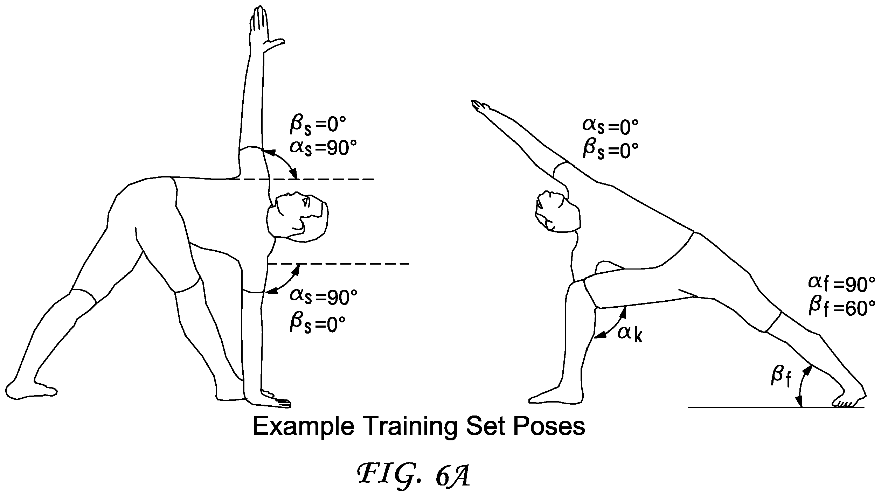

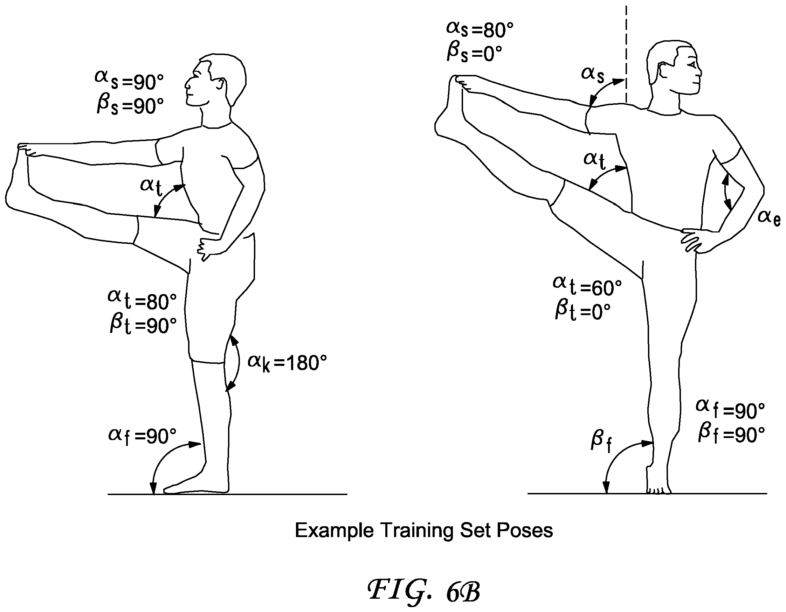

Figures 6a and 6b provide example yoga poses to be performed by the user as training for the model 330. The poses include various angles that can be determined via the sensor set in a wearable item. The Figures 6a and 6b show some of the angle values defined in the example PoseVector definition of Table 3 in Figure 10.

In one aspect of the disclosure, when new unknown sensors are detected during the registration process, the system would enter the "training" mode. The training mode involves several steps as explained below. Note that this is a one-time process for each new type of sensor. First, the information about the type of sensor, the parameter it measures, and the range of measurements are gathered. This can be done automatically using some standard communication protocols or manually by a user entering information from the manufacturer of the smart clothing. Then the training system gathers some physical information about user's body (such as size, height, weight, etc). This can also be automated using, for example, a scale (for weight) and image processing methods for size of body parts. For each pose the data acquisition system gathers information from all sensors. The data acquisition system provides the training system with a set of SensorVectors (processed sensor data) captured for each pose.

A data processing algorithm receives the PoseId (pose identifier) and user's physical data and calculates the corresponding PoseVector (pose data) representing the pose for the VR environment (such as body part sizes and angles). A machine learning algorithm is used to train a model based on the labeled training samples. Each sample in the training set is in the form of a pair such as (SensorVector, PoseVector) (processed sensor data, pose data). After the training of the model is complete, the model will be able to receive any SensorVector and generate its corresponding PoseVector. The training information is then saved to the system and will be used for other users using the same smart clothing item. Several different machine learning algorithms can be used for training the model using the training samples. (Support Vector Machines, Neural-Networks, Gradient boosting, etc.)

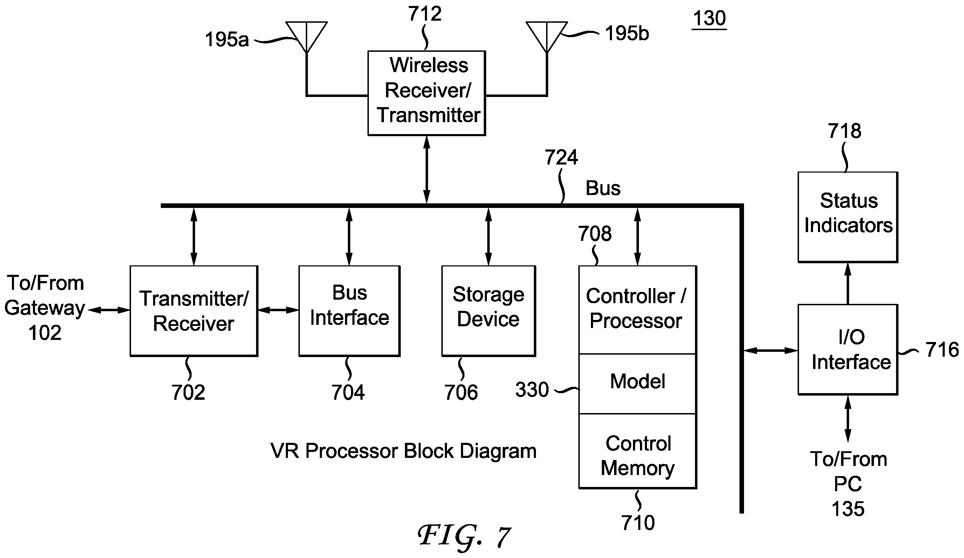

Figure 7 is an example embodiment of a VR processor, such as item 130 of Figures 1 and 2. Here, a connection to a gateway 120 is via the transmitter/receiver interface 702. The gateway interface 702 connects to the bus interface 704 which allows access to the internal bus 724. Other non-bus implementations are also possible as is well known to those of skill in the art. Present on bus 724 are a storage device 706 which can be used for any general storage such as retrieved or requested data and network management data, parameters, and programs. Storage device 706 may also serve as disk or solid-state storage for the user profile information, machine learning, and machine model used for conversion of SensorVector to PoseVector. Machine learning training data may also be stored in storage 706. Such utility and other programs are under the control of controller/processor 708. Storage 706 can also be removeable, such as by a CD, DVD, Solid State, or other technology known in the art, and be capable of instruction storage to perform the method of Figure 5.

The controller/processor 708 may be a single processor or a multiplicity of processors performing the tasks of vector conversion, user interface control, and resource managements. Controller/processor 708 can also perform machine learning training for the VR processor 130. However, in one embodiment, since the training of the model is a one-time process, training can be accomplished offline on a more powerful external computer system (with multiple CPUs and GPUs) and the trained model information be transferred to this computer system 130. In any event, a trained machine learning model is executed on controller/processor 708 to provide the conversion between SensorVector data (processed sensor data) and the PoseVector data (pose data).

Control memory 710 can supply program instruction and configuration control for controller/processor 708. The status indicators are a user interface 718 and allows a user, system owner, or system manager to see a status of the VR Processor 130. Such indicators may include a display, LEDs, printer interface, or data logging interface. An input/output (I/O) interface 716 allows the VR Processor 130 to connect to a personal computer or other device that can be used to configure and control the VR functionality. The I/O interface 716 may be a hardline interface, such as an Ethernet interface or may operationally be substituted with an RF interface so that the VR Processor 130 can communicate with a PC via a protocol driven interface, such as IEEE 802.XX. Alternately, a remote terminal, such as PC 135 may also be connected to a WLAN. Other interfaces that are possible via I/O interface 716 are an interactive interface which may include the use of a display device, keyboard, mouse, light pen, and the like.

VR Processor 130 has a wireless network interface 712 which allows access to and from the sensor sets 150, 170 and VR viewers 140 and 160. Such an interface includes all elements to control a wireless network, including the use of wireless network protocols such as IEEE 802.XX and the like. The wireless network interface includes a receiver to receive raw sensor data, convert to SensorVecor data (processed sensor data) and a transmitter to transmit PoseVector information (pose vector) for display. The display (not shown in Figure 7) renders the body pose, such as may be displayed on a VR display. The controller/processor 708 of the VR Processor 130 of Figure 2 is configured to provide processing services for the steps of the methods of Figure 5. For example, the controller processor can provide instruction control to monitor and control the Gateway interface 702, the I/O interface 716 and 718, and the WLAN interface 712. Controller/processor 708 directs the flow of information through VR Processor 130 such that the method step activities of Figure 5 is performed.

In one embodiment, a method to render a body pose of a user includes acquiring sensor data from a plurality of sensors worn by a user, processing the acquired sensor data to generate a processed sensor data set, wherein the processed sensor data set is scaled for the size of the user, converting the processed sensor data set into a pose data set, translating the pose data set to a format compatible with a viewer, and transmitting the formatted pose data set to the viewer that renders the body pose of the user.

In the embodiment, acquiring sensor data includes acquiring an identifier and value pair for each of the plurality of sensors. Acquiring sensor data from a plurality of sensors can include acquiring sensor data from a garment worn by the user of a virtual reality system or an augmented reality system. Acquiring sensor data can include acquiring sensor data from one or more of a strain sensor, pressure sensor, magnetometer, accelerometer, and level indicator. Processing the acquired sensor data can include applying one of a time-tag or a numerical index to each processed sensor data set. Converting the processed sensor data set into a pose data set can include correlating processed sensor data with pose data. The embodiment can further include interpolating pose data from processed sensor data that do not exactly match correlated processed sensor data. Acquiring sensor data from a plurality of sensors can include periodically receiving measurements from sensors worn over a body of the user. Transmitting the formatted pose data set to a viewer that renders the body pose of the user can include transmitting formatted body pose information to other users in a virtual reality system or an augmented reality system.

In one embodiment, an apparatus to provide information for a body pose of a user includes a receiver for receiving sensor data from a plurality of sensors worn by the user, a processor for processing the received plurality of sensor data, the processor is configured to provide a processed sensor data set, wherein the processed sensor data set is scaled to the size of the user, and wherein the processor is configured to convert the processed sensor data set into a pose data set, and includes a transmitter for providing the pose data set to a viewer in a format that can render the body pose of the user.

In the embodiment, the receiver is configured to receive data from one or more of the plurality of sensors including one or more of a strain sensor, a pressure sensor, a magnetometer, an accelerometer, and a level indicator. The processor is configured to acquire multiple sets of sensor data, to provide multiple sets of processed sensor data, and to apply one of a time-tag or a numerical index to each of the processed sensor data sets. The processor can include a machine learning model for calculation of the body pose data set from the processed sensor data set. The machine learning model is can be trained using one of the processor and an external computer system. The processor is configured to translate the pose data set to a format compatible with the viewer. The receiver can be a wireless receiver.

Any or all of the features described herein may be combined into a single embodiment unless otherwise specifically stated. The implementations described herein may be implemented in, for example, a method or process, an apparatus, or a combination of hardware and software. Even if only discussed in the context of a single form of implementation (for example, discussed only as a method), the implementation of features discussed may also be implemented in other forms. For example, implementation can be accomplished via a hardware apparatus, hardware and software apparatus. An apparatus may be implemented in, for example, appropriate hardware, software, and firmware. The methods may be implemented in, for example, an apparatus such as, for example, a processor, which refers to any processing device, including, for example, a computer, a microprocessor, an integrated circuit, or a programmable logic device. Multiple processors may also be used in place of or in addition to the controller/processor shown in Figure 7.

Additionally, the methods may be implemented by instructions being performed by a processor, and such instructions may be stored on a processor or computer-readable media such as, for example, an integrated circuit, a software carrier or other storage device such as, for example, a hard disk, a compact diskette ("CD" or "DVD"), a random-access memory ("RAM"), a read-only memory ("ROM") or any other magnetic, optical, or solid state media. The instructions may form an application program tangibly embodied on a computer-readable medium such as any of the media listed above or known to those of skill in the art. The instructions thus stored are useful to execute elements of hardware and software to perform the steps of the method described herein. Thus, a computer readable medium, such as one adaptable to interface with storage device 706 in Figure 7, may be used to execute instructions to perform the method of Figure 5. In addition, a computer program product is contemplated that has instructions thereon which when executed by one or more processors. The instructions, upon execution, cause the one or more processors to carry out the method of Figure 5.

## Claims

1. A method of providing body pose information, the method comprising:
acquiring sensor data from a plurality of sensors worn by a user;
processing the acquired sensor data to generate a processed sensor data set, wherein the processed sensor data set is scaled for the size of the user;
converting the processed sensor data set into a pose data set;
translating the pose data set to a format compatible with a viewer; and
transmitting the formatted pose data set to the viewer that renders a body pose of the user.

2. The method of claim 1, wherein acquiring sensor data comprises acquiring an identifier and value pair for each of the plurality of sensors.

3. The method of any of claims 1-2, wherein acquiring sensor data from a plurality of sensors comprises acquiring sensor data from a garment worn by the user of a virtual reality system or an augmented reality system.

4. The method of any of claims 1-3, wherein acquiring sensor data comprises acquiring sensor data from one or more of a strain sensor, pressure sensor, magnetometer, accelerometer, and level indicator.

5. The method of any of claims 1-4, wherein processing the acquired sensor data comprises applying one of a time-tag or a numerical index to each processed sensor data set.

6. The method of any claims 1-5, wherein converting the processed sensor data set into a pose data set comprises correlating processed sensor data with pose data.

7. The method of claim 6, further comprising interpolating pose data from processed sensor data that do not exactly match correlated processed sensor data.

8. The method of any of claims 1-7, wherein acquiring sensor data from a plurality of sensors comprises periodically receiving measurements from sensors worn over a body of the user.

9. The method of any claims 1-8, wherein transmitting the formatted pose data set to the viewer that renders the body pose of the user comprises transmitting formatted body pose information to other users in a virtual reality system or an augmented reality system.

10. An apparatus for providing information for a body pose of a user, the apparatus comprising:
a receiver for receiving sensor data from a plurality of sensors worn by the user;
a processor for processing the received plurality of sensor data, the processor is configured to provide a processed sensor data set, wherein the processed sensor data set is scaled to the size of the user, and wherein the processor is configured to convert the processed sensor data set into a pose data set;
a transmitter for providing the pose data set to a viewer in a format that can render the body pose of the user.

11. The apparatus of claim 10, wherein the receiver is configured to receive data from one or more of the plurality of sensors comprising one or more of a strain sensor, a pressure sensor, a magnetometer, an accelerometer, and a level indicator.

12. The apparatus of any of claims 10-11, wherein the processor is configured to acquire multiple sets of sensor data, to provide multiple sets of processed sensor data, and to apply one of a time-tag or a numerical index to each of the processed sensor data sets.

13. The apparatus of any of claims 10-12, wherein the processor includes a machine learning model for calculation of the body pose data set from the processed sensor data set.

14. The apparatus of any of claims 10-13, wherein the processor is configured to translate the pose data set to a format compatible with the viewer.

15. A computer program product comprising instructions which when executed by one or more processors cause the one or more processors to carry out the method of any one of claims 1-9.
